# EUROPEAN PATENT SPECIFICATION

(11) **EP 0 842 801 B1**
(45) Date of publication and mention of the grant of the patent: **21.08.2002**
(21) Application number: 96308284.7
(22) Date of filing: 15.11.1996
(51) Int. Cl.: B60H 3/00, A61L 9/12

(54) **Accessory structure for vehicle air-conditioner**
Zusatzvorrichtung für eine Fahrzeugklimaanlage
Accessoire pour un climatiseur de véhicule

(43) Date of publication of application: 20.05.1998
(73) Proprietor: Akazawa, Akira, Matsubara, Osaka (JP)
(72) Inventor: Akazawa, Yasumasa, (JP)
(74) Representative: Warren, Keith Stanley

(56) References cited:
- US-A- 4 890 791
- US-A- 4 913 034
- US-A- 5 060 858
- US-A- 5 078 046
- US-A- 5 297 988

## Description

The present invention relates to an accessory structure for a vehicle air-conditioner, and in particular to a structure for improving the atmosphere of the interior or compartment of a vehicle by feeding functional solvents into the air-conditioner for cleaning the heat exchanger.

To this end, a solvent spray nozzle is provided at the upstream side of the heat exchanger in the air-conditioning air intake route. A hose communicating with this nozzle leads out into the compartment, and a solvent container is installed in a case near the driver's seat or front seat of the vehicle. A base end of the hose is fitted to the solvent discharge port of the filled container. However, locating the filled container near the driver's seat or front seat may be dangerous, since the contents of the container are discharged using a gas propellant, and the filled container may be ruptured or the gas may be overheated by exposure to direct sunlight or by the elevation of the compartment temperature.

A vehicle air-conditioning system is described in Patent US 4,913,034 (on which the preamble of claim 1 is based). The system includes a deodorising liquid reservoir and a pump which are situated remotely from an air-conditioning unit including a blower, a heater and an evaporator unit. The pump is arranged to pump deodorising liquid from the reservoir to a discharge nozzle situated closely downstream of the blower so that it can pass through the heater or evaporator unit depending on the position of a diverter flap valve situated downstream of the discharge nozzle. The discharge nozzle is accordingly situated remotely from the evaporator unit. It is an object of the present invention to provide an accessory structure for a vehicle air-conditioner which addresses the problems discussed above and provides efficient cleaning of an evaporator situated in such an air-conditioner.

According to the present invention there is provided a vehicle air-conditioner and an accessory structure therefor, the vehicle air-conditioner comprising
means for taking in external air or internal air for passage through an air intake route and
a heat exchanger for cooling input air,
and the accessory structure comprising:
at least one solvent flow injection means located at the upstream side of the heat exchanger in the air intake route,
characterised by
a solvent feed route having a leading end which is fixed to the injection means and a base end which is feedable into a vehicle interior for feeding solvent to the solvent flow injection means from a solvent source, and a solvent receiving means which is separable from said solvent source and coupled to the base end of the solvent feed route, said receiving means being locatable in a vehicle interior, for supplying solvent into the solvent feed route when solvent is flowing from a solvent source to the injection means.

It is an advantage of the invention that efficient cleaning of the evaporator situated in the air-conditioner is effected.

It is an advantage of an embodiment of the invention that the accessory structure is capable of feeding the solvent adequately, by providing the receiving means with a guide for positioning the nozzle of the solvent source when feeding the solvent.

It is a further advantage of an embodiment of the invention that the accessory structure is capable of improving controllability, by forming a taper in the guide, so that the nozzle may be guided into the optimum position for feeding solvent, by the taper, if the nozzle of the solvent source is brought close to the guide from an oblique direction.

It is a further advantage of an embodiment of the invention that the accessory structure is capable of preventing dust and foreign matter from sticking to or invading the solvent passage of the guide, by disposing closing means, for closing the solvent passage of the guide, in the inner side of a lid member for covering the guide.

Embodiments of the present invention will now be described, by way of example, with reference to the accompanying drawings, in which:
Fig 1 is a side view showing an accessory structure in accordance with the present invention;
Fig 2 is a perspective view of the nozzle of Fig 1;
Fig 3 is a sectional view of a receiving member in accordance with the present invention;
Fig 4 is a plan view of the receiving member of Fig 3 without a lid;
Fig 5 is an explanatory diagram of the lid member of Fig 3;
Fig 6 is an explanatory diagram showing the feeding of a solvent;
Fig 7 is an explanatory diagram showing the use of foamy cleaning fluid in the accessory structure;
Fig 8 is a sectional view showing another embodiment of a receiving member in accordance with the present invention; and
Fig 9 is a sectional view showing a different embodiment of a receiving member in accordance with the present invention.

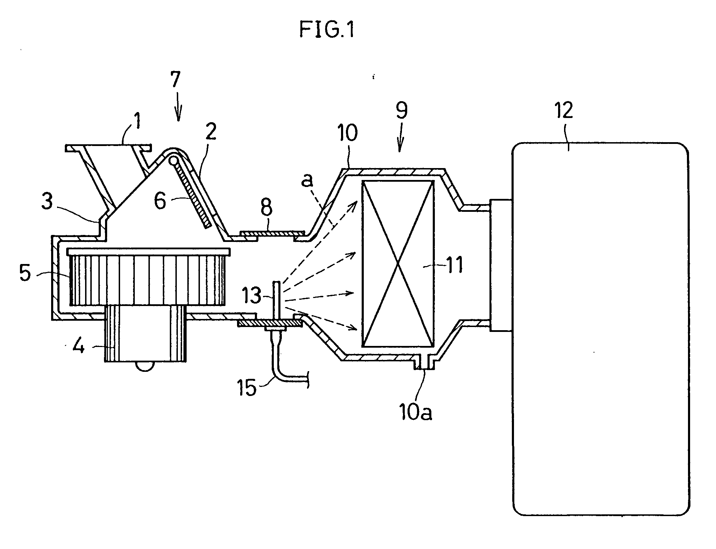

The drawings show an accessory structure for a vehicle air-conditioner,and first describe the structure of the vehicle air-conditioner, as illustrated in Fig 1. An internal/external air changeover box 3 having an external air intake port 1 and an internal air intake port 2 is provided. A fan 5 driven by a blower motor 4 is also disposed in the box 3, together with an internal/external air changeover door 6 for selecting the source of the air taken into the air-conditioner.

In a next. stage, a cooler unit 9 is disposed adjacent the blower unit 7, and connected to it via a communication duct 8. The cooler unit 9 incorporates an evaporator 11 1 as the heat exchanger, in a cooler housing 10. A drain port 10a is formed in the cooler housing 10 immediately beneath the evaporator 11.

The evaporator 11 is an evaporating device connected in a refrigerating cycle, and acts to draw heat from the surrounding.

In a next stage a heater control unit 12 is connected to the cooler unit 9, remote from the blower unit 7. Inside this unit 12, there is provided a heater core, an air mixing door, a vent door, a defroster door, a heat door,and a mixing chamber, and by changeover of these doors, air-conditioned air (cool air or warm air) is blown out into the required positions in the interior of the vehicle from the defroster outlet, vent outlet, and heat outlet (not shown).

In the vehicle air-conditioner the communication duct 8 positioned at the upstream side of the evaporator 11 is provided with a nozzle 13 for injecting a solvent fluid (a) toward nearly the entire region of the front side of the evaporator 11.

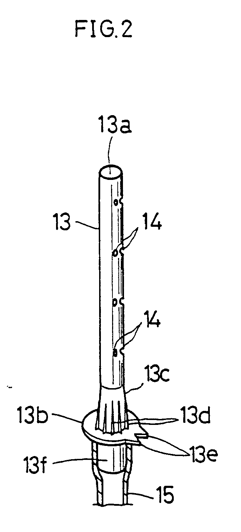

The nozzle 13 is a relatively slender cylindrical form made of synthetic resin having a closed leading end 13a as shown in Fig 2. Sets of two injection ports 14, are pierced in the front surface of the evaporator 11 as indicated by the dotted line arrows in Fig 1, illustrating a possible passage of solvent out of the ports.

A nozzle neck 13c near a flange 13b is formed in a taper tube, the smaller diameter end of which is the leading end 13a, and the larger diameter end of which is located at the flange 13b. Also, plural, fluted protrusions 13d of triangular cross section, to be inserted and stopped in the rubber communication duct, are formed integrally on the outer circumference of the nozzle 13. Accordingly, by merely piercing pores in the communication duct 8, the nozzle 13 can be mounted instantly and securely. Of course, bonding means for bonding the flange 13b to the communication duct 8 may also be employed.

Furthermore, in the flange 13b, a protrusion 13e, for marking at the time of mounting the tube in the duct 8 and nearly coinciding with the injection direction of the solvent fluid (a), is formed integrally, so that the nozzle 13 may be mounted in the correct orientation. The leading end of the rubber hose 15 (solvent feed route) is adhered and fixed to a joint 13f in the nozzle 13 by making use of its elastic tightening force (elastic restoring force) i.e. the hose 15 is pushed over the nozzle joint 13f.

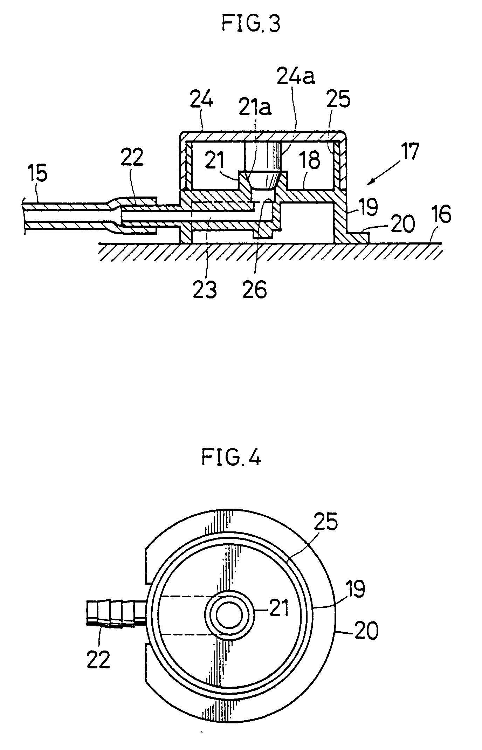

The base end side of the rubber hose 15 is fed into the vehicle interior and is coupled to a receiving member 17 made of a synthetic resin. The member 17 acts as a receiving means and is fixed at a specific place near the driver's seat or front seat as shown in Fig 3, for example, in a specific plate at the front, side or bottom of an instrument panel 16 by proper means (adhering, bolting, clipping, or taping with double-sided adhesive tape).

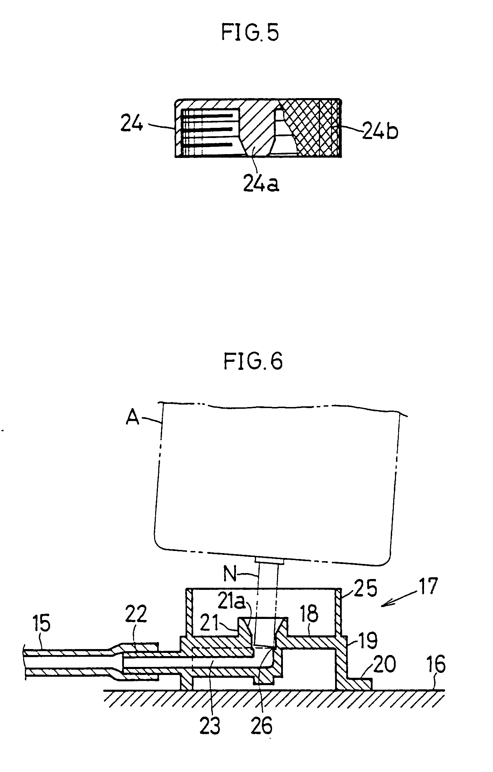

The receiving member 17 is completely separable and isolatable from a filled container A of solvent which acts as a solvent source (see Fig 6), and its specific constitution is as shown in Fig 3 to Fig 6.

That is, a flange 20 is integrally formed in a main plate 18 through a ring-shaped spacer 19, and a solvent passage 23 is formed between a guide 21 of a nearly cylindrical form provided in the middle of the main plate 18 and a joint 22 projecting outward from part of the outer circumference of the spacer 19,while a ring-shaped holder 25 for supporting a lid member 24 is integrally formed in the main plate 18, thereby composing the main body side of the receiving member 17.

The guide 21 is intended to position the nozzle N (see Fig 6) of the filled container A (a hand held container packed with gas) to be inserted when feeding the solvent, and a taper 21a larger in diameter at the outward side and smaller in diameter at the inward side is formed in the guide 21, and an annular step 26 for preventing excessive insertion of the nozzle N is formed at the inner side of the taper 21a.

The lid member 24 is detachably screwed into the holder 25. Inside the lid member 24, a protrusion 24a is provided, as closing means, for closing the solvent passage 23 of the guide 21. More specifically, the taper 21 a is formed integrally with the main plate 18, and the outer circumference of the lid member 24 is roughly processed so as to facilitate attachment and detachment of the member 24. In Fig 5, as an example of roughening processing, a knurled portion 24b is shown, but other roughening processes may also be used.

The outer shape of the joint 22 is formed in detent shape having a multiple tapered cone structure as shown in Fig 4, and the base end of the rubber hose 15 is fitted and fixed to the joint 22, by effectively utilising its elastic tightening force.

In this way, by completely separating and isolating the receiving member 17 from the hand held container A, the filled container A can be put in the glove box or console box of the vehicle, when not in use.

Solvents which are suitable for use include liquid detergent, foamy cleaning solution (water, phosphoric acid, Softanol 70, propylene glycol monomethyl ether MGF, and others properly blended), cleaning water, aromatics, chemicals, deodorant, deodorizer, disinfectant, antibacterial bactericide, and fungicide, which may be used either alone or may be mixed (compound). For aromatic effect in the compartment a filled container packed with aromatic solvent may be used.

Elements, 13, 14, 15 and 17 shown in Figs 2 and 3 are handled as a kit, and the elements 13 to 15 and 17 may additionally be attached to the vehicle, or may be formed integrally when manufacturing the vehicle.

The action of the accessory structure for a vehicle air-conditioner thus formed is described below.

When cleaning the evaporator 11, after detaching the lid member 24 from the receiving member 17 shown in Fig 3, the nozzle N of the filled container A is inserted into the solvent passage 23 while being guided by the taper 21a of the guide 21 as shown in Fig 6, and the cleaning fluid is pressed out from the nozzle N, and then the cleaning fluid is supplied into the rubber hose 15 through the solvent passage 23 and flows into the nozzle 13 from the leading end, and is injected from multiple injection ports 14 towards nearly the entire area of the front surface of the evaporator 11. Thus the complicated arrangement of fins (not shown) of the evaporator can be cleaned almost entirely, and the fluid, after the cleaning process, is discharged from the drain port 10a immediately beneath the evaporator 11. If necessary, a drain hose may be connected to the drain port 10a, and the fluid may be discharged out of the vehicle.

By driving the fan 5 while injecting the cleaning fluid from the injection ports 14, the cleaning fluid reaches further into the inner parts of the fins so that the cleaning effect may be further enhanced.

The sequence of use of solvent fluids may be as follows: foamy detergent and chemical such as disinfectant or antibacterial, or foamy detergent,cleaning water, and chemical or liquid detergent and antibacterial, and moreover after the cleaning process, the evaporator 11 may be dried by feeding air (warm air, hot air or cold air) from the receiving member end of the rubber hose 15.

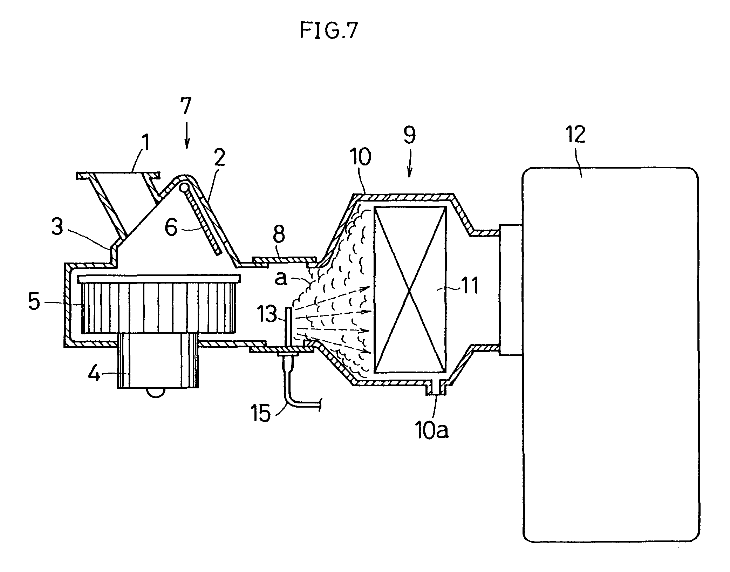

In particular, when the solvent fluid injected from the nozzle 13 is a foamy cleaning fluid (a), if the dimension of the nozzle 13 is relatively short as compared with the internal overall height of the communication duct 8 as shown in Fig 7, the foamy cleaning fluid injected from the nozzle 13 is spread over the entire front region (that is, full surface) of the upstream side of the evaporator 11 as shown in Fig 7 owing to the characteristic of the foamy fluid, and the entire evaporator 11 can be cleaned efficiently, including its inner parts, by the foamy cleaning fluid by circulation of air-conditioning air. Therefore, the nozzle 13 may be compact in size, and mounting of the nozzle 13 on the vehicle air-conditioner may be further eased.

In short, the rubber hose 15 is connected to the nozzle 13 provided at the upstream side of the evaporator 11 in the air-conditioning air intake route (the route from air intake ports 1, 2 to the evaporator 11), the receiving member 17, separated from the solvent A is provided at the receiving member end of the rubber hose 15, and the receiving member 17 is fixed in a specific place in the compartment. Therefore the solvent source, such as a filled container A is separated from the receiving member 17 and can be put in an accommodating space, for example, a glove box or console box, where it is not heated to a high temperature, so that rupture or damage of the solvent source may be prevented. At the time of use, the solvent container A is taken out from the accommodation space, and is inserted into the nozzle guide, and the evaporator 11 is cleaned, or aromatic or other functional solvent is supplied, so that the compartment atmosphere may be improved.

Further, since the receiving member 17 is provided with the guide 21 for positioning the nozzle N of the filled container A when feeding the solvent, the solvent can be supplied appropriately by positioning the nozzle N of the filled container at the guide 21 of the receiving member 17.

Moreover, since the taper 21a is formed in the guide 21, if the nozzle N of the filled container A is brought closer to the guide, this nozzle is guided into the solvent feed in an optimum position by the taper 21a, so that controllability may be enhanced.

In addition, the protrusion 24a for closing the solvent passage 23 of the guide 21 provided inside the lid member 24 for covering at least the guide 21 is effective to prevent dust and other foreign matter from sticking to or invading the solvent passage 23 of the guide 21.

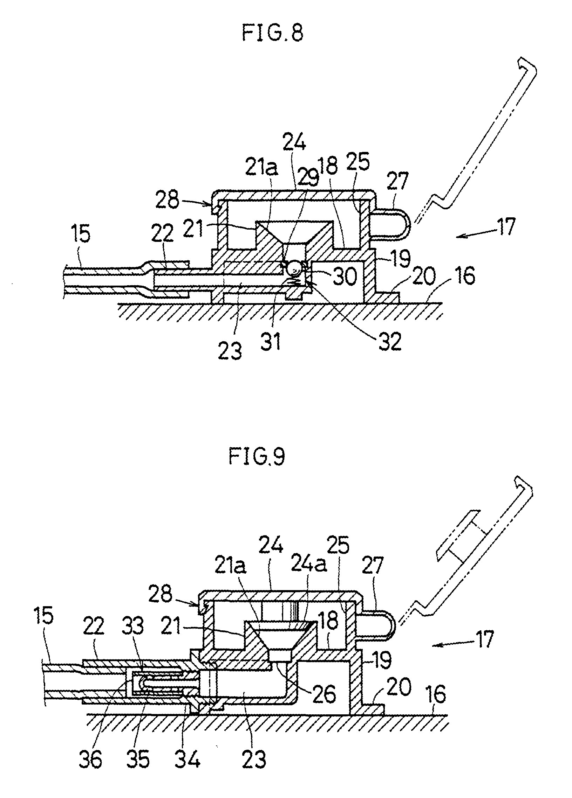

Fig 8 shows another embodiment of the receiving member 17, in which the lid member 24 is integrally connected to the ring-shaped holder 25 through an elastic piece 27 in a lateral U form. These members 24,25 are attachable or detachable instantly using cooperating convex and concave fixing means 28. The convex portion -is formed in the lid member 24 and the concave portion formed in the holder 25,thereby enhancing the controllability when closing the lid member 24 and preventing loss of the member 24 when opening the lid member 24.

Still further, in an intermediate portion of the solvent passage 23, a check valve 32 comprising a valve seat 29, a ball valve 30,and a spring 31 having the same function as the annular step 26 are disposed so as to prevent counterflow of the solvent fluid (a).

Thus, since the check valve 32 is provided in the solvent passage 23 between the guide 21 and the base end portion of the rubber hose 15, counterflow of the solvent can be securely blocked.

In the embodiment of Fig 8, too, the other members have the same function and effect as in the foregoing embodiment, and the same parts as in the preceding drawings are identified with same reference numerals in Fig 8.

Fig 9 shows a further different embodiment of the receiving member 17, in which the joint 22 is larger in diameter than the one shown in the foregoing embodiments, and is detachable from the spacer 19, the joint 22 being screw-threaded for attachment to the spacer 17. A counterflow preventive member 33 is detachably disposed in the joint 22.

The counterflow preventive member 33 comprises a pipe member 34 screwed into the joint 22 and closed at the leading end, plural openings 35 pierced in the pipe member 34 for circulating the solvent, and a tube member 36 fitted to the outer circumference of the pipe member 35 and bulging and deforming by the solvent pressure to allow circulation of solvent. Thus, when the solvent pressure is released, the openings 35 are closed by contracting by the restoring force of the material of the tube member 36, thereby preventing counterflow.

In this embodiment, the receiving member end portion of the rubber hose 15 is connected to communicate inside the joint 22.

In this way, since the counterflow preventive member 33 is disposed in the solvent passage between the guide 21 and receiving member end portion of the rubber hose 15, counterflow of the solvent can be blocked securely.

In the embodiment in Fig 9, too, the other members have the same action and effect as in the foregoing embodiments, and same parts as in the preceding drawings are identified with same reference numerals in Fig 9.

In correspondence between the formation of the accessory structure and the foregoing embodiments, air intake route corresponds to the route from the air intake ports 1,2 to the evaporator 11 in the embodiments, and similarly thereafter, the heat exchanger, to the evaporator 11, the solvent injection means, to the nozzle 13, the solvent feed route, to the rubber hose 15, the solvent source, to the filled container A, the receiving means, to the receiving member 17, the counterflow preventive means, to the check valve 32, or the counterflow preventive member 33, and the closing means, to the protrusion 24, but it must be noted, however, the invention is not limited to the illustrated embodiments alone.

For example, in Fig 1 and Fig 7, the evaporator 11 is disposed in the next stage to the fan 5 in the vehicle air-conditioner, but in the vehicle air-conditioner of the type in which the fan is disposed in the evaporator, the nozzle 13 may be provided at the upstream side of the evaporator, so as not to interfere with the rotary locus of the internal/external air changeover door.

In the embodiments, only a set of constituent elements composed of elements 13 to 15 and 17 was used, but using plural sets of constituent elements, the nozzles 13 may additionally be disposed at different positions at the upstream side of the evaporator 11 and upstream side of the fan 5, and the elements 15 and 17 may be used independently for each solvent.

Moreover, the solvent feed route may be, instead of rubber hose 15, resin tube or metal piping, and the structure of the nozzle 13 is not limited to the illustrated embodiments alone.

## Claims

1. A vehicle air-conditioner and an accessory structure therefor, the vehicle air-conditioner comprising
means (1, 2, 6) for taking in external air or internal air for passage through an air intake route and
a heat exchanger (11) for cooling input air, and the accessory structure comprising:
at least one solvent flow injection means (13) located at the upstream side of the heat exchanger (11) in the air intake route,
**characterised by**
a solvent feed route (15) having a leading end which is fixed to the injection means (13) and a base end which is feedable into a vehicle interior for feeding solvent to the solvent flow injection means (13) from a solvent source (A), and a solvent receiving means (17) which is separable from said solvent source (A) and coupled to the base end of the solvent feed route (15), said receiving means (15) being locatable in a vehicle interior, for supplying solvent into the solvent feed route when solvent is flowing from a solvent source (A) to the injection means (13).

2. An air-conditioner and accessory structure as claimed in claim 1 wherein the receiving means (17) comprises a guide (21) for positioning a nozzle (N) of the solvent source (A) when feeding solvent into the air-conditioner.

3. An air-conditioner and accessory structure as claimed in claim 2 wherein the guide (21) is tapered with a larger diameter at its outward side and a smaller diameter at its inward side.

4. An air-conditioner and accessory structure as claimed in claim 2 or 3 comprising solvent counterflow preventive means (30-33) disposed between the guide (21) and the receiving means end portion of the solvent feed route (15).

5. An air-conditioner and accessory structure as claimed in any preceding claim wherein the receiving means (17) has a lid member (24) for covering at least the guide means (21), and closing means (24a), for closing the solvent passage (23) of the guide (21), formed inside of the lid member (24).

6. An air-conditioner and accessory structure as claimed in any preceding claim wherein the solvent receiving means (17) is attachable to a surface in the vehicle interior.

## Patentansprüche

1. Fahrzeugklimaanlage und eine Zubehörkonstruktion dafür, wobei die Fahrzeugklimaanlage folgendes umfasst:
Mittel (1, 2, 6) zum Ansaugen von Außenluft oder Innenluft zum Strömen durch einen Lufteinlassweg, und
einen Wärmetauscher (11) zum Kühlen von Eingangsluft, wobei die Zubehörkonstruktion folgendes umfasst:
wenigstens ein Lösungsmittelinjektionsmittel (13), das dem Wärmetauscher (11) im Lufteinlassweg vorgeschaltet ist, **gekennzeichnet durch**:
eine Lösungsmittelzuleitung (15) mit einem vorderen Ende, das an dem Injektionsmittel (13) befestigt ist, und einem Basisende, das ins Fahrzeuginnere geführt werden kann, um Lösungsmittel von einer Lösungsmittelquelle (A) zum Lösungsmittelinjektionsmittel (13) zu führen, und ein Lösungsmittelaufnahmemittel (17), das von der genannten Lösungsmittelquelle (A) getrennt und mit dem Basisende der Lösungsmittelzuleitung (15) gekoppelt werden kann, wobei das genannte Aufnahmemittel (15) im Inneren eines Fahrzeugs positioniert werden kann, um Lösungsmittel in die Lösungsmittelzuleitung zu speisen, wenn Lösungsmittel von einer Lösungsmittelquelle (A) in das Injektionsmittel (13) fließt.

2. Klimaanlage und Zubehörkonstruktion nach Anspruch 1, wobei das Empfangsmittel (17) eine Führung (21) zum Positionieren einer Düse (N) der Lösungsmittelquelle (A) während des Speisens von Lösungsmittel in die Klimaanlage umfasst.

3. Klimaanlage und Zubehörkonstruktion nach Anspruch 2, wobei sich die Führung (21) von einem größeren Durchmesser auf ihrer Außenseite auf einen kleineren Durchmesser auf ihrer Innenseite verjüngt.

4. Klimaanlage und Zubehörkonstruktion nach Anspruch 2 oder 3, umfassend Mittel (30-33) zum Verhindern eines Lösungsmittelrückflusses, die zwischen der Führung (21) und dem Aufnahmemittel-Endabschnitt der Lösungsmittelzuleitung (15) angeordnet sind.

5. Klimaanlage und Zubehörkonstruktion nach einem der vorherigen Ansprüche, wobei das Aufnahmemittel (17) ein Deckelelement (24) zum Abdecken wenigstens das Führungselementes (21) und ein innerhalb des Deckelelementes (24) ausgebildetes Verschlussmittel (24a) zum Verschließen des Lösungsmittelkanals (23) der Führung (21) umfasst.

6. Klimaanlage und Zubehörkonstruktion nach einem der vorherigen Ansprüche, wobei das Lösungsmittelaufnahmemittel (17) an einer Oberfläche im Fahrzeuginneren befestigt werden kann.

## Revendications

1. Un climatiseur pour véhicule et une structure accessoire pour celui-ci, le climatiseur pour véhicule comportant
des moyens (1, 2, 6) pour capter de l'air extérieur ou de l'air intérieur pour le faire passer à travers un circuit d'admission d'air et
un échangeur de chaleur (11) pour refroidir l'air introduit, et la structure accessoire comprenant :
au moins un moyen d'injection d'un débit de solvant (13) installé du côté amont de l'échangeur de chaleur (11) dans le circuit d'admission d'air, **caractérisé par**
un circuit d'alimentation en solvant (15) qui a une extrémité antérieure qui est fixée au moyen d'injection (13) et une extrémité de base qui peut être introduite dans l'intérieur d'un véhicule pour alimenter en solvant le moyen d'injection de débit de solvant (13) à partir d'une source de solvant (A), et un moyen récepteur de solvant (17) qui est séparable de ladite source de solvant (A) et qui est couplé à l'extrémité de base du circuit d'alimentation en solvant (15), ledit moyen récepteur (15) pouvant être installé dans l'intérieur d'un véhicule, pour fournir du solvant dans le circuit d'alimentation en solvant lorsque le solvant est débité par une source de solvant (A) jusqu'au moyen d'injection (13).

2. Un climatiseur et une structure accessoire selon la revendication 1, dans lesquels le moyen récepteur (17) comprend un guide (21) pour positionner un distributeur (N) de la source de solvant (A) lorsque le solvant est envoyé dans le climatiseur.

3. Un climatiseur et une structure accessoire selon la revendication 2, dans lesquels le guide (21) est conique et présente un diamètre plus grand sur son côté extérieur et un diamètre plus petit sur son côté intérieur.

4. Un climatiseur et une structure accessoire selon la revendication 2 ou 3, comprenant des moyens qui évitent les retours de solvant (30-33) disposés entre le guide (21) et la partie d'extrémité du moyen récepteur de le circuit d'alimentation en solvant (15).

5. Un climatiseur et une structure accessoire selon l'une quelconque des revendications précédentes, dans lesquels le moyen récepteur (17) a un couvercle (24) pour couvrir au moins le moyen de guidage (21), et un moyen de fermeture (24a) pour fermer le passage de solvant (23) du guide (21), formé à l'intérieur du couvercle (24).

6. Un climatiseur et une structure accessoire selon l'une quelconque des revendications précédentes, dans lesquels le moyen récepteur de solvant (17) peut être fixé à une surface à l'intérieur du véhicule
